(19) Europäisches Patentamt
European Patent Office
Office européen des brevets

(11) EP 2 463 319 A2

(12) **EUROPEAN PATENT APPLICATION**
published in accordance with Art. 153(4) EPC

(43) Date of publication:
**13.06.2012 Bulletin 2012/24**

(21) Application number: **10806630.9**

(22) Date of filing: **02.08.2010**

(51) Int Cl.:
***C08F 255/00*** *(2006.01)* ***C08F 265/06*** *(2006.01)*
***C08F 2/04*** *(2006.01)* ***C07C 329/00*** *(2006.01)*

(86) International application number:
**PCT/KR2010/005077**

(87) International publication number:
**WO 2011/016656 (10.02.2011 Gazette 2011/06)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **07.08.2009 KR 20090072879**

(71) Applicant: **Hong, Sung Chul**
**Munjeong 1-dong Songpa-gu**
**Seoul 138-764 (KR)**

(72) Inventors:
- **HONG, Sung Chul**
  **Seoul 138-764 (KR)**
- **JOO, Sang Il**
  **Cheonan-si**
  **Chungcheongnam-do 331-170 (KR)**

(74) Representative: **Zeestraten, Albertus W. J.**
**Exter Polak & Charlouis B.V. (EP&C)**
**P.O. Box 3241**
**2280 GE Rijswijk (NL)**

# (54) METHOD FOR PREPARING OLEFIN-BASED SEGMENTED COPOLYMERS

(57) The present application relates to a method for preparing olefin-based segmented copolymers through reversible addition-fragmentation chain transfer (RAFT), wherein the method comprises: a step of polymerizing vinyl-based monomers through reversible addition-fragmentation chain transfer (RAFT) using a trithiocarbonate-based chain transfer agent to synthesize vinyl-based polymers; and a step of grafting the synthesized vinyl-based polymers to olefin-based polymers. The method of the present invention successfully synthesizes olefin-based segmented copolymers through the two simple above-described steps.

[FIG. 6]

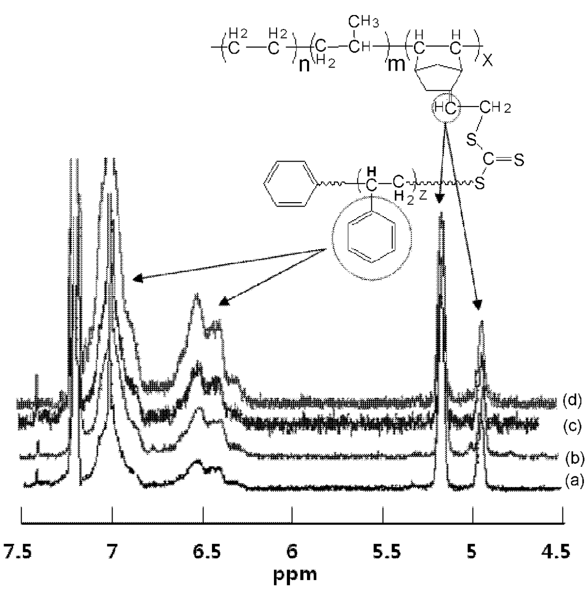


EP 2 463 319 A2

## Description

### Technical Field

The present invention relates to a method of preparing olefin-based segmented copolymers using reversible addition-fragmentation chain transfer (RAFT), and more particularly to a method of preparing olefin-based segmented copolymers, which may synthesize a vinyl-based polymer using a trithiocarbonate-based chain transfer agent upon RAFT so that such a vinyl-based polymer is easily grafted onto an olefin-based polymer.

### Background Art

Research into controlling the molecular structures of polymers to adjust chemical and physical properties thereof is very important in the field of polymer chemistry. The polymer chains are classified into homopolymers and copolymers depending on the components thereof, and also into a variety of forms depending on the type of connection of chains. Changes in these components and molecular structures result in chemical and physical properties of polymers being modified, and thus polymers having a variety of properties may be obtained via synthesis of appropriate polymers. Conventional research into modifying polymer structures is of interest because an original structure of a polymer is modified, thereby exhibiting specific properties different from the original properties of the polymer, and such properties may be efficiently used. For example, when an olefin polymer chain which is mainly used industrially is substituted with a hydroxyl group or a carbonyl group, it may become hydrophilic or polar. As such, the modification proportion or amount of the chain is regarded as important to achieve a modification purpose. If the degree of modification is not high, original properties of the polymer are not greatly changed. In addition to the properties, however, adhesion or compatibility with insoluble materials may be improved. Also, cross-linking of linear polymer materials may change viscosity, glass transition temperature, etc., of polymers.

The bonding of heterogeneous polymer components is mainly focused on binding components having supplementary properties to each other, and block copolymers and graft copolymers may be obtained via conventional chemical bonding of a main polymer chain and a heterogeneous polymer. The block copolymer is obtained by polymerizing a heterogeneous monomer to the terminal of the chain, and living polymerization is chiefly utilized. The graft copolymer is obtained by polymerizing a heterogeneous polymer using an initiation point on the repeating unit of the chain (*grafting-from*), or by making a heterogeneous polymer chain on the main chain using a heterogeneous polymer and a functional group of the main chain (*grafting-onto*).

Modification of olefin polymers have been studied for a long time. Research into graft copolymers has been carried out in terms of polar polymers being grafted onto polyethylene (PE), polypropylene (PP), ethylene-propylene-diene terpolymers (EPDM), etc. Modification of olefin polymers to prepare graft copolymers is initiated by removing hydrogen from the olefin polymer chain using peroxide, followed by polymerizing a heterogeneous polymer. However, initiation efficiency of olefin polymer chains is low and it is difficult to obtain a desired graft copolymer from only the bonding of initiated chains. Thus, there is a need for research into effectively grafting a heterogeneous polymer.

Recent research is ongoing into modification of olefin polymers, diene polymers, etc., using controlled/living radical polymerization (CRP) which is advantageous because the synthesis of a polymer having a designed structure is possible thanks to efficient control of polymerization reaction of a polymer. Typical radical polymerization leads to non-uniform molecular weight and a wide molecular weight distribution. The living radical polymerization which is under active study in recent years controls the chain transfer or stopping reaction during the polymerization reaction, thus facilitating adjustment of the molecular weight and molecular weight distribution, structure design of the polymer chain and introduction of the functional group, and control of the copolymer composition, whereby such a polymerization method is frequently applied to increasing functionality and performance of materials and to preparation of novel polymer materials.

However, concrete methods have not yet been devised for efficiently and simply preparing olefin-based segmented copolymers using reversible addition-fragmentation chain transfer (RAFT).

## Disclosure

### Technical Problem

Accordingly, the present invention has been made keeping in mind the above problems occurring in the related art, and an object of the present invention is to provide a novel method of preparing olefin-based segmented copolymers, wherein not only the length and number of graft chains of the olefin-based segmented copolymer but also the grafting efficiency may be adjusted, thus providing a new technical platform of living radical polymerization for modifying a polymer.

## Technical Solution

In order to accomplish the above object, a first aspect of the present invention provides a method of preparing an olefin-based segmented copolymer, comprising polymerizing a vinyl-based monomer via RAFT using a trithiocarbonate-based chain transfer agent to synthesize a vinyl-based polymer; and grafting the synthesized vinyl-based polymer onto an olefin-based polymer.

A second aspect of the present invention provides an olefin-based segmented copolymer prepared using the above method.

## Description of Drawings

FIG. 1 is of graphs showing the conversion of styrene to a polymer and an increase in the molecular weight according to an embodiment of the present invention;

FIG. 2 is of graphs showing the conversion of styrene to a polymer and an increase in the molecular weight under conditions different from those of FIG. 1, according to an embodiment of the present invention;

FIG. 3 is a graph showing FT-IR spectrum of EPDM and PS;

FIG. 4 is a graph showing the calibration curve determined by calculating the peak height ratio relative to the amount of PS after physically mixing EPDM and PS;

FIG. 5 is a graph showing FT-IR spectrum of PS-grafted EPDM according to an embodiment of the present invention;

FIG. 6 is a graph showing $^1$H-NMR spectrum of PS-grafted EPDM according to an embodiment of the present invention;

FIG. 7 is a graph showing GPC results of EPDM before and after a grafting reaction according to an embodiment of the present invention; and

FIG. 8 shows the synthesis scheme of EPDM-graft-PS using melting reaction according to an embodiment of the present invention.

## Mode for Invention

Hereinafter, embodiments and examples of the present invention are described in detail with reference to the appended drawings so as to be easily performed by a person having ordinary skill in the art.

The following description does not limit the present invention to specific embodiments, and should be understood to include all variations, equivalents or substitutions within the spirit and scope of the present invention. Furthermore, descriptions of known techniques, even if they are pertinent to the present invention, are considered unnecessary and may be omitted in so far as they would make the characteristics of the invention unclear.

The terms used herein are merely intended to explain specific examples and not to limit the present invention. Unless otherwise stated, the singular expression includes a plural expression. In this application, the terms "include" or "have" are used to designate the presence of features, numbers, steps, operations, elements, parts or combinations thereof described in the specification, and should be understood so as not to exclude the presence or additional probability of one or more different features, numbers, steps, operations, elements, parts or combinations thereof.

As used herein, the segmented copolymer means a copolymer having different polymer segments, and is construed to include a graft copolymer and a block copolymer, and more preferably it is meant to a graft copolymer.

A first aspect of the present invention provides a method of preparing an olefin-based segmented copolymer comprising polymerizing a vinyl-based monomer via RAFT (Reversible Addition-Fragmentation Chain Transfer) using a trithiocarbonate-based chain transfer agent (or RAFT agent) to synthesize a vinyl-based polymer (first step); and grafting the synthesized vinyl-based polymer onto an olefin-based polymer (second step).

In the first step, a polymer which is to be grafted in order to impart the olefin-based polymer with desired properties is prepared. Particularly, a polymer which is to be grafted is synthesized using a trithiocarbonate-based chain transfer agent having a trithiocarbonate unit in lieu of a typical dithioester chain transfer agent When polymerization is carried out using such a trithiocarbonate-based chain transfer agent in this way, the resulting polymer has a trithiocarbonate unit therein. The following grafting procedure of the second step may be performed using the polymer thus produced.

As mentioned above, the first step is used to polymerize the vinyl-based monomer via RAFT using a trithiocarbonate-based chain transfer agent, thus synthesizing the vinyl-based polymer. The schematic reaction thereof is represented by Scheme 1 below.

[Scheme 1]

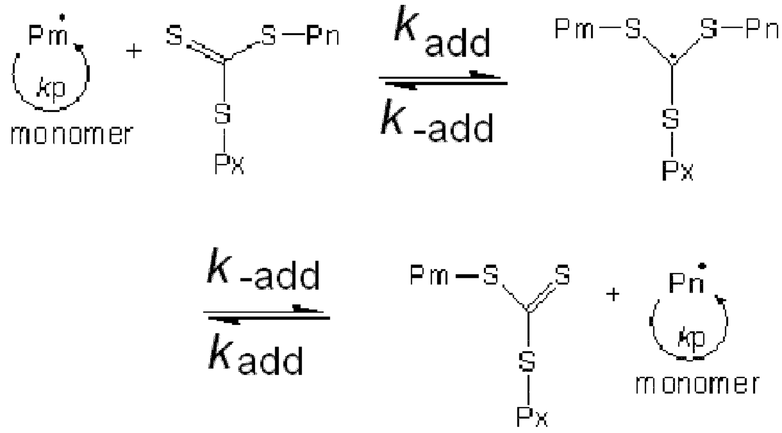


The trithiocarbonate-based chain transfer agent is referred to as a compound that has a trithiocarbonate unit in the molecule thereof and may be used as a chain transfer agent in the RAFT reaction, and preferably is compound represented by Chemical Formula 1 below, but is not limited thereto.

[Chemical Formula 1]

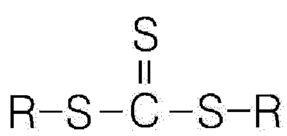

In Chemical Formula 1, two Rs are respectively independently selected from the group consisting of alkyl; alkenyl; saturated, unsaturated or aromatic carbocyclic or heterocyclic ring; alkylthio; alkoxy; and dialkylamino, and the alkyl; alkenyl; saturated, unsaturated or aromatic carbocyclic or heterocyclic ring; alkylthio; alkoxy; and dialkylamino may be independently substituted with a substituent selected from the group consisting of epoxy, hydroxy, alkoxy, aryl, acyl, acyloxy, carboxy and salts thereof, sulfonic acid and salts thereof, alkylcarbonyloxy, isocyanato, cyano, silyl, halo and dialkylamino.

The alkyl is preferably $C_1$~$C_{18}$ alkyl, and more preferably $C_1$~$C_6$ alkyl, and the carbocyclic ring or heterocyclic ring preferably has 5~14 ring atoms, but is not limited thereto.

The vinyl-based monomer is a vinylic monomer which is free-radical polymerizable, and preferably includes a compound represented by Chemical Formula 2 below and a combination thereof, but is not limited thereto.

[Chemical Formula 2] $CH_2$=CUV

In Chemical Formula 2, U is selected from the group consisting of hydrogen; halogen; and $C_1$~$C_4$ alkyl which may be substituted with a substituent independently selected from the group consisting of hydroxy, $C_1$~$C_{18}$ alkoxy, aryloxy (OR'), carboxy, acyloxy, aroyloxy ($O_2CR'$), alkoxy-carbonyl and aryloxy-carbonyl ($CO_2R'$), and

V is selected from the group consisting of hydrogen, R', $CO_2H$, $CO_2R'$, COR', CN, $CONH_2$, CONHR', $CONR'_2$, $O_2CR'$, OR' and halogen,

wherein R' is independently selected from the group consisting of $C_1$~$C_{18}$ alkyl, $C_2$~$C_{18}$ alkenyl, aryl, heterocyclyl, aralkyl and alkaryl, and the $C_1$~$C_{18}$ alkyl, $C_2$~$C_{18}$ alkenyl, aryl, heterocyclyl, aralkyl, and alkaryl may be substituted with a substituent independently selected from the group consisting of epoxy, hydroxy, alkoxy, aryl, acyl, acyloxy, carboxy and salts thereof, sulfonic acid and salts thereof, alkoxy- or aryloxy-carbonyl, isocyanato, cyano, silyl, halo, and dialkylamino.

According to an embodiment of the present invention, the vinyl-based monomer may be selected from the group consisting of methyl methacrylate, ethyl methacrylate, propyl methacrylate, butyl methacrylate, 2-ethylhexyl methacrylate, isobornyl methacrylate, methacrylic acid, benzyl methacrylate, phenyl methacrylate, methacrylonitrile, alpha-methylstyrene, methyl acrylate, ethyl acrylate, propyl acrylate, butyl acrylate, 2-ethylhexyl acrylate, isobornyl acrylate, acrylic acid, benzyl acrylate, phenyl acrylate, acrylonitrile, styrene; functionalized methacrylate, acrylate and styrene selected from the group consisting of glycidyl methacrylate, 2-hydroxyethyl methacrylate, hydropropyl methacrylate,

hydroxybutyl methacrylate, N,N-dimethylaminoethyl methacrylate, N,N-diethylaminoethyl methacrylate, triethyleneglycol methacrylate, itaconic anhydride, itaconic acid, glycidyl acrylate, 2-hydroxyethyl acrylate, hydroxypropyl acrylate, hydroxybutyl acrylate, N,N-dimethylaminoethyl acrylate, N,N-diethylaminoethyl acrylate, triethyleneglycol acrylate, methacrylamide, N-methylacrylamide, N,N-dimethylacrylamide, N-tert-butylmethacrylamide, N-n-butylmethacrylamide, N-methylolmethacrylamide, N-ethylolmethacrylamide, N-tert-butylacrylamide, N-n-butylacrylamide, N-methylolacrylamide, N-ethylolacrylamide, vinyl benzoic acid, diethylaminostyrene, alpha-methylvinyl benzoic acid, diethylamino alphamethylstyrene, p-vinylbenzene sulfonic acid, sodium p-vinylbenzenesulfonate, trimethoxysilylpropyl methacrylate, triethoxysilylpropyl methacrylate, tributoxysilylpropyl methacrylate, dimethoxymethylsilylpropyl methacrylate, diethoxymethylsilylpropyl methacrylate, dibutoxymethylsilylpropyl methacrylate, diisopropoxymethylsilylpropyl methacrylate, dimethoxysilylpropyl methacrylate, diethoxysilylpropyl methacrylate, dibutoxysilylpropyl methacrylate, diisopropoxysilylpropyl methacrylate, trimethoxysilylpropyl acrylate, triethoxysilylpropyl acrylate, tributoxysilylpropyl acrylate, dimethoxymethylsilylpropyl acrylate, diethoxymethylsilylpropyl acrylate, dibutoxymethylsilylpropyl acrylate, diisopropoxymethylsilylpropyl acrylate, dimethoxysilylpropyl acrylate, diethoxysilylpropyl acrylate, dibutoxysilylpropyl acrylate, diisopropoxysilylpropyl acrylate, vinyl acetate, vinyl butyrate, vinyl benzoate, vinyl chloride, vinyl fluoride, vinyl bromide, maleic anhydride, N-phenylmaleimide, N-butylmaleimide, N-vinylpyrrolidone, N-vinylcarbazole, butadiene, isoprene, chloroprene and propylene; and combinations thereof, but is not limited thereto.

According to an embodiment of the present invention, the vinyl-based polymer synthesized by polymerizing the vinyl-based monomer may be selected from the group consisting of polystyrene, polyacrylate, polymethacrylate, and polyacrylonitrile, but is not limited thereto.

In addition to the trithiocarbonate-based chain transfer agent and the vinyl-based monomer mentioned in the first step, a radical initiator may be further added, as necessary, and the radical initiator may initiate the polymerization of the vinyl-based monomer, and any radical initiator may be used without particular limitation so long as it is typically used for RAFT polymerization.

In the second step, the olefin-based polymer and the vinyl-based polymer that is synthesized in the first step are reacted, thereby grafting the vinyl-based polymer onto the chain of the olefin-based polymer. Preferably the second step may be carried out in the presence of a radical generator that generates reactive radical sites on the olefin-based polymer. When the olefin-based polymer and the synthesized vinyl-based polymer are reacted in the presence of the radical generator, the vinyl-based polymer having the trithiocarbonate unit functions as a chain transfer agent due to radicals generated on the olefin-based polymer and is thus grafted onto the olefin-based polymer. As such, the radical generator is not particularly limited so long as it generates reactive radical sites on the olefin-based polymer.

The olefin polymer includes a homopolymer and a copolymer resulting from polymerization of an olefin monomer. According to an embodiment of the present invention, the olefin polymer may be selected from the group consisting of polyethylene, polypropylene, polybutadiene, an ethylene-propylene-diene terpolymer (EPDM), and combinations thereof, but is not limited thereto.

The second step may be carried out in a solution phase in a solvent. According to an embodiment of the present invention, the solvent may be for example xylene, but is not limited thereto and a variety of organic solvent may be used. Also, the second step may be conducted in a melt phase instead of using the solvent.

A second aspect of the present invention provides an olefin-based segmented copolymer prepared by the above method. The preparation method according to the present invention enables the production of copolymers wherein the vinyl-based polymer is grafted onto the olefin-based polymer such as polyethylene, polypropylene, polybutadiene, an ethylene-propylene-diene terpolymer, etc., which are industrially mainly used, and also enables the modification to desired polymers having improved properties and thus may be applied to a variety of fields.

The following examples are set to illustrate the present invention but are not construed as limiting the present invention.

[Example]

<Reagent>

As an olefin polymer, ethylene-propylene-diene terpolymer (EPDM) rubber, KEP-650L ($M_n$ = 120700 g/mol, $M_w/M_n$ = 2.20, ethylidene norbornene content = 8.9 wt%, about 90 ethylidene norbornene units per EPDM chain) available from Kumhopolychem was used. As a chain transfer agent, dibenzyl trithiocarbonate (DBTTC) was available from Arkema. Sytrene (99.8wt%, Aldrich) was vacuum distilled under $CaH_2$ and then used. 2,2-azobisisobutyronitrile (AIBN, 98 wt%, Samchun Chemical), dicumyl peroxide (DCP, 99 wt%, Aldrich), xylene (98 wt%, Samchun Chemical), acetone (95 wt%, Samchun Chemical), tetrahydrofuran (THF, 98 wt%, Samchun Chemical) and anisole (99 wt%, Aldrich) were used without additional purification.

<Analysis Method>

Gel Permeation Chromatography (GPC) Analysis

GPC was performed using RI-detector and UV-detector (RID-10A, SPD-20AV, Shimadzu) and three columns (Styragel HR 5, 4, 2). The molecular weight was calibrated using a polystyrene standard, and was measured by injecting 40 $\mu\ell$ of a sample using a HPLC THF solvent under conditions of an oven temperature of 40°C, a pump pressure of 5 MPa, and a flow rate of 1.0 mL/min.

Gas Chromatography (GC) Analysis

Used as the column of GC (GC-2010, Shimadzu) was VB-WAX (length 30 m, inner diameter 0.32 mm, film thickness 0.25 $\mu$m), and the measurement conditions of the column were a temperature of 40□ and an equilibration time of 1.5 min. The detector was FID1 at 250□, and the injection mode was split, and the sampling time was 1 min, and $N_2$, $H_2$ and air having a purity of 99.99% were used at a pressure of 48.3 kPa, a total flow of 83.8 mL/min, a purge flow of 3.0 mL/min, and a column flow of 1.58 mL/min. The conditions of gas flow were $N_2$ =30 mL/min, $H_2$ =40 mL/min, and air = 400 mL/min.

FT-IR and $^1$H-NMR analysis

Fourier transform infrared spectroscopy (FT-IR) was performed using Thermo nicolet 380. The sample for FT-IR was prepared in such a manner that in the case of PS, a sample dissolved in THF was dropped onto a KBr window and then dried in a vacuum oven for 12 hours or longer, and in the case of EPDM, a film was prepared using a hot press. $^1$H-NMR spectroscopy was performed using a 500 MHz Bruker Avance spectrometer. The solvent used was d-CDCl, and a peak by TMS (tetramethylsilane) was used as a standard peak.

1. Polymerization of Polystyrene (PS) using DBTTC

Polymerization was carried out using a 200 mL Schlenk flask. The reaction temperature was maintained using an oil bath on a heater the temperature of which was adjustable, and a nitrogen atmosphere in the reactor was maintained. Typical polymerization procedures for obtaining PS were as follows.

[Scheme 2]

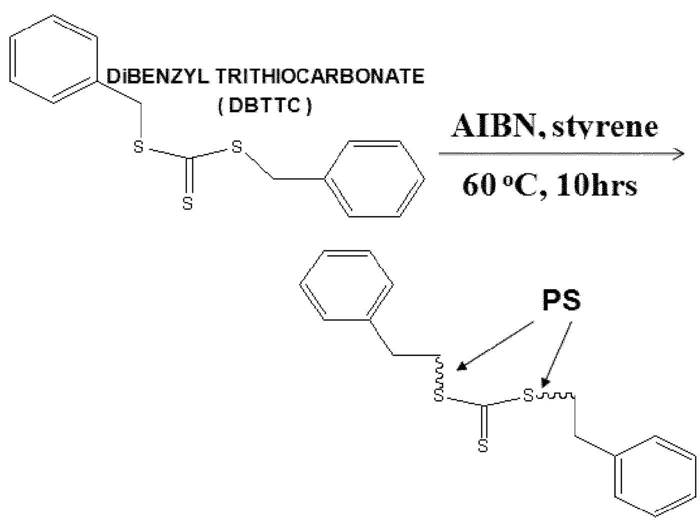


Styrene (48 mL, 0.42 mol), AIBN (0.42 g, 2.6 x $10^{-3}$ mol), DBTTC (0.65 mL, 2.33 x $10^{-3}$ mol), and anisole (10% v/v based on styrene, added as a standard material for GC monitoring of polymerization reaction) were added into a Schlenk flask together with a magnetic stirrer and a nitrogen atmosphere was maintained ([Sty]:[AIBN]:[DBTTC] = 180: 1.1:1). Three freeze-pump-thaw cycles were performed to remove oxygen, and the mixture was stirred at 60□ and 350 rpm to polymerize it. The resulting product was precipitated using methanol, and dried in a vacuum oven at 60□ for 24 hours. During the polymerization reaction, the sample was taken at intervals of 0, 1, 2, 3, 4 and 5 hours, and the

conversion of styrene into a polymer, the number average molecular weight, the theoretical molecular weight and the molecular weight distribution were measured using GC and GPC.

2. Synthesis of EPDM-graft PS via *graftinq-onto* reaction of prepared PS (Solution Reaction)

The reaction was carried out using a 100 mL Schlenk flask. The reaction temperature was maintained using an oil bath on a heater the temperature of which was adjustable, and a nitrogen atmosphere in the reactor was maintained. Typical synthesis procedures for obtaining EPDM-graft-PS were as follows.

[Scheme 3]

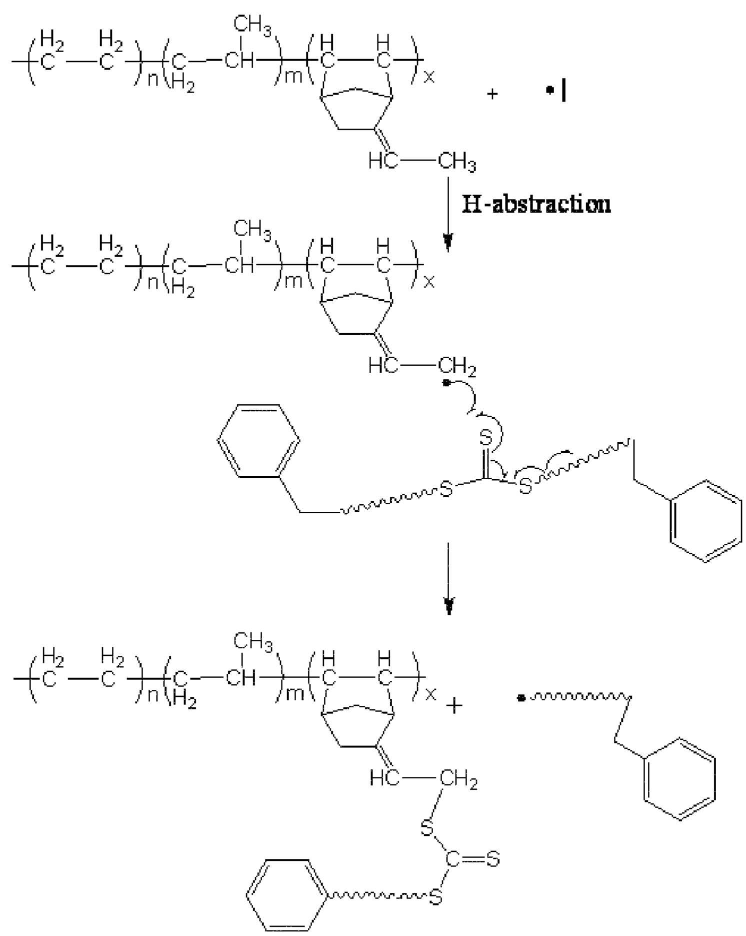


EPDM (1 g, 8.0 x $10^{-6}$ mol), PS (0.96 g, 1.85 x $10^{-3}$ mol), dicumyl peroxide (DCP, 0.05 g, 1.85 x $10^{-3}$ mol), and xylene (40 mL) were added into a Schlenk flask, and stirred using a magnetic stirrer to dissolve them. After EPDM was completely dissolved in xylene, $N_2$ bubbling was conducted for 30 min ([PS]:[DCP] = 1:1). The mixture was reacted with stirring at 138□ for 4 hours at 350 rpm. As such, the reaction time was set to allow sufficient decomposition in consideration of the half time of DCP (1 hour at 138□). After the reaction, unreacted PS was extracted using acetone, dissolved in a THF solution and then precipitated again in acetone. The resulting product was dried in a vacuum oven at 40□ for 10 hours, and analyzed using FT-IR and $^1$H-NMR. Respective test conditions are shown in Table 1 below.

[TABLE 1]

| Name | EPDM (g) | PS (g) | PS (recipe, wt%) | DCP (g) |
|---|---|---|---|---|
| sE-S01 | 1 | 0.48 | 32.43 | 0.05 |
| sE-S02 | 1 | 0.48 | 32.43 | 0.1 |
| sE-S03 | 1 | 0.96 | 48.99 | 0.05 |

(continued)

| Name | EPDM (g) | PS (g) | PS (recipe, wt%) | DCP (g) |
|---|---|---|---|---|
| sE-S04 | 1 | 0.96 | 48.99 | 0.1 |

3. Synthesis of EPDM-qraft-PS via *grafting-onto* reaction of prepared PS (Melting Reaction)

In order to prepare EPDM-graft PS using a melting reaction, an internal mixer (Brabender Plasticorder PLE 331) was used. Typical procedures were described below (FIG. 8). EPDM (140 g, 1.2 x $10^{-3}$ mol) and prepared PS (15 g, 2.5 x $10^{-3}$ mol) were placed into the internal mixer, heated to 100□ (resin temperature: 120˚C), and then kneaded at a rate of a rotor of 20 rpm. The mixture was mixed for 5 min, added with dibenzoyl peroxide (0.6 g, 2.5 x $10^{-3}$ mol) and then reacted for 20 min ([PS]:[di-benzoyl peroxide] = 1:1). The reaction time was set to allow sufficient decomposition in consideration of the half time of dibenzoyl peroxide (1 min at 100□). The unreacted PS of the reactants was extracted using acetone as an extraction solution by means of a Soxhlet extractor for 24 hours, and the final product was dried in a vacuum oven at 40□for 10 hours and then analyzed using FT-IR to ascertain the structure thereof. Respective test conditions are shown in Table 2 below.

[TABLE 2]

| Name | EPDM (g) | PS (g) | PS (recipe, wt%) | DBP[b](g) |
|---|---|---|---|---|
| mE-S01 | 140 | 15 | 9.7 | - |
| mE-S02 | 140 | 15 | 9.7 | 0.6 |
| mE-S03 | 140 | 15 | 9.7 | 1.2 |
| mE-S04 | 140 | 15 | 9.7 | 2.4 |

4. Analysis Results

Polymerization of PS using DBTTC

In order to prepare EPDM-gmfi-PS via the "*grafting-onto*" reaction as mentioned above, a graft chain, that is, PS was polymerized using DBTTC as a chain transfer agent (a RAFT agent).

FIG. 1 shows the conversion of styrene into a polymer over time and an increase in molecular weight of PS with respect to the conversion of styrene ([Sty]:[AIBN]:[DBTTC] = 180:1.1 :1, $[\text{styrene}]_0$ = 7.90 mol/L, temperature = 60□). As shown in FIG. 1a, the conversion of styrene was linearly increased over time. FIG. 1b shows a linear increase in the number average molecular weight with respect to the conversion of styrene, and the molecular weight distribution is shown to be ~ 1.2. Accordingly, the polymerization could be seen to be successfully carried out in the type of living polymerization. The theoretical molecular weight in FIG. 1b was calculated from the following equation, and was not significantly different from the actual molecular weight.

$$M_{n,th} = M_{DBTTC} + [(\text{conversion of styrene}) \times [\text{styrene}]_0/([\text{DBTTC}]_0 + [\text{AIBN}]_0 \times (1 - e^{-kini \times t}))] \times M_{styrene}$$

In the above equation, $M_{DBTTC}$ (296 g/mol) and $M_{styrene}$ (104.15 g/mol) are molecular weights of DBTTC and styrene, respectively, and $[\text{styrene}]_0$, $[\text{DBTTC}]_0$ and $[\text{AIBN}]_0$ are initial concentrations of styrene monomer, DBTTC and AIBN, respectively, and $e^{-kini}$ is the constant of decomposition of AIBN represented by 0.9 L/mol·min, and t is the polymerization time.

Living polymerization capable of adjusting the molecular weight was applied and the amount of added styrene and the reaction time were changed, so that polymerization was carried out. FIG. 2 shows the conversion of styrene into a polymer over time and an increase in the molecular weight of PS with respect to the conversion of styrene under changed conditions ([Sty]:[AIBN]:[DBTTC] = 955:1.1:1, $[\text{styrene}]_0$ = 7.90 mol/L, temperature = 80□). As shown in FIG. 2, the number average molecular weight was linearly increased with respect to the conversion of styrene, and the molecular weight distribution of ~1.2 was shown, so that the polymerization was carried out in the type of living polymerization. Thereby, living radical polymerization was successfully achieved resulting in adjusted molecular weight and

molecular weight distribution. Respective test conditions, molecular weight and molecular weight distribution are shown in Table 3 below.

[TABLE 3]

| | Styrene (mL) | AIBN (g) | DBTTC (mL) | Temp.(˚C) | Time | $M_n$ | $M_w/M_n$ |
|---|---|---|---|---|---|---|---|
| PS 01 | 48 | 0.42 | 0.65 | 60 | 5 | 5900 | 1.17 |
| PS 02 | 255 | 0.42 | 0.65 | 80 | 10 | 23400 | 1.19 |

Synthesis of EPDM-qraft-PS using *grafting-onto* reaction of prepared PS (Solution Reaction)

As mentioned above, the test of grafting the trithiocarbonate unit of PS polymerized using a chain transfer agent (DBTTC) onto EPDM was carried out. Xylene was used as a reaction solvent, and dicumyl peroxide (DCP) for removing hydrogen from EPDM was added along with PS polymerized from DBTTC and then the mixture was reacted at a predetermined temperature. While the amounts of PS and peroxide were changed under respective conditions, the results were observed.

When EPDM and PS are reacted in the presence of peroxide as shown in Scheme 3, PS having the trithiocarbonate unit plays a role as a chain transfer agent due to the radicals generated on EPDM and is thus grafted onto EPDM. As such, the PS having the trithiocarbonate unit had adjusted molecular weight and molecular weight distribution. Using a $^1$NMR analysis method, quantitative analysis of branched PS per EPDM chain upon grafting is possible.

Although quantitative analysis was possible using $^1$H-NMR, when the rubber was partially cured due to heat or peroxide upon reaction, it was not dissolved in a solvent, making it impossible to perform analysis. For this reason, quantitative analysis using FT-IR was attempted.

FIG. 3 shows FT-IR results of EPDM and PS. The specific peaks of EPDM (C-C stretches, 1160 $cm^{-1}$) and PS (aromatic C=C stretches (four bands), 1590 $cm^{-1}$) by FT-IR were observed. In FIG. 4, EPDM and PS were physically mixed and the peak height ratio relative to the amount of PS was calculated and represented by the calibration curve. As such, the peak height ratio relative to the amount of PS was shown to be tendency, from which the degree of branching of the product was estimated.

The FT-IR results of PS-grafted EPDM are shown in FIG. 5 (sE-S01 (a), sE-S02 (b), sE-S03 (c), sE-S04 (d), and the sample compositions and names are shown in Table 4 below), and the $^1$H-NMR results of PS-grafted EPDM are shown in FIG. 6. As shown in FIG. 6, the peaks of aromatic protons of PS were observed in the range of 6.3 ~ 7.5 ppm, and the peaks of protons of =CH- in the ethyldiene norbornene unit of EPDM were observed at 4.9 ppm and 5.2 ppm. The molecular weight (Mn =120700) of EPDM was confirmed by GPC analysis and the number of ethyldiene norbornene units (90 units/chain) was calculated from the amount of ethyldiene norbornene (ENB content = 8.9 wt%) of the EPDM chain. On the assumption that the unreacted PS was completely extracted, the degree of branching of PS was calculated from the integral value of the peaks of aromatic protons of PS and the proton peaks of =CH- in the ethyldiene norbornene unit of EPDM. Table 4 below shows the weight of PS grafted onto EPDM and the number of chains as analyzed using FT-IR and $^1$H-NMR depending on the test compositions.

[TABLE 4]

| | EPDM (g) | PS (g) | PS (recipe, wt%) | DCP (g) | PS (FT-IR, wt%) | Number of PS Chains (FT-IR) | PS ($^1$H-NMR, wt%) | Number of PS Chains ($^1$H-NMR) |
|---|---|---|---|---|---|---|---|---|
| sE-S01 | 1 | 0.48 | 32.43 | 0.05 | 1.19 | 2.52 | 1.9 | 0.78 |
| sE-S02 | 1 | 0.48 | 32.43 | 0.1 | 1.27 | 2.68 | 4.73 | 1.94 |
| sE-S03 | 1 | 0.96 | 48.99 | 0.05 | 1.90 | 4.03 | 5.35 | 2.19 |
| sE-S04 | 1 | 0.96 | 48.99 | 0.1 | 3.39 | 7.16 | 11.64 | 4.77 |

Table 5 below shows the grafting efficiency (GE) based on quantitative analysis results of PS grafted onto EPDM shown in Table 4.

[TABLE 5]

| | PS (FT-IR, wt%)[a] | Number of PS Chains (FT-IR) | GE (FT-IR, %) | PS ($^1$H-NMR, wt%)[b] | Number of PS Chains ($^1$H-NMR) | GE ($^1$H-NMR, %) |
|---|---|---|---|---|---|---|
| sE-S01 | 1.19 | 2.52 | 7.77 | 1.9 | 0.78 | 5.95 |
| sE-S02 | 1.27 | 2.68 | 8.26 | 4.73 | 1.94 | 14.59 |
| sE-S03 | 1.90 | 4.03 | 8.23 | 5.35 | 2.19 | 10.92 |
| sE-S04 | 3.39 | 7.16 | 14.62 | 11.64 | 4.77 | 23.75 |

The grafting efficiency (GE) was calculated from the following equation.

$$Grafting\ efficiency\,(\%) = \frac{weight\ of\ grafted\ PS}{weight\ of\ PS\ used\ for\ reaction} \times 100$$

In the above equation, the weight of PS used for the reaction was determined by the test composition, and the weight of grafted PS was determined by FT-IR and $^1$H-NMR results.

As is apparent from Tables 4 and 5, the number of sites wherein PS may be branched in the chain of EPDM was increased in proportion to an increase in the amount of peroxide, and thus PS was estimated to be much more branched. When the amount of PS was increased, the amount of chain transfer agent became enriched upon reaction, making it possible to branch a larger amount of PS.

As is apparent from the grafting efficiency of sE-S02 and sE-S03 calculated via FT-IR and $^1$H-NMR analysis of Table 5, sE-S02 having the composition wherein the amount of PS is 1/2 and the amount of peroxide is double compared to sE-S03 is observed to have lowered grafting efficiency. Hence, there is a need for research into the efficiency of forming a radical by removing hydrogen from the chain of EPDM by a peroxide and into a chain transfer reaction of PS having a chain transfer agent unit to the radical of the chain of EPDM, and there is required to precisely control the kind and amount of used peroxide and the amount of PS having the chain transfer agent unit.

FIG. 7 shows GPC results of EPDM before and after the "*grafting-onto*" reaction. When comparing the GPC curve before polymerization with the GPC curve after polymerization, the curve is seen to have shifted.

Synthesis of EPDM-graft PS using qraftinq-onto reaction of prepared PS (Melting Reaction)

The solution reaction may cause environmental problems, cost problems and mass production problems attributed to the use of a solvent. In order to solve the problems of such a wet reaction, a melting reaction has been studied. This does not use the solvent unlike the wet reaction and allows modification materials to react in an internal mixer. The test composition shown in Table 2 was used. As results of mixing at different temperatures, when the temperature of the mixer was 100□ and the temperature of modification materials was 120□, the materials were observed to be well mixed with the naked eye. When the temperature of the mixer was 100□, the product was dissolved in a THF solution, from which partial curing was observed to occur during the reaction procedure. The degree of curing increased in proportion to an increase in the amount of peroxide and a decrease in the amount of PS. This is considered to be because when the amount of PS is increased the amount of the trithiocarbonate unit acting as a chain transfer agent is increased to thus prevent the cross-linking of EPDM chains. In order to remove unreacted PS after the reaction, unreacted PS was extracted by means of a Soxhlet extractor using acetone as an extraction solution for 24 hours. The final product was dried and analyzed with FT-IR to ascertain the structure and the degree of branching. As results of FT-IR, the specific peaks of PS were also observed in EPDM-graft PS prepared using the melting reaction as in the solution reaction, from which PS was evaluated to be branched.

As mentioned above, the ethylene-propylene-diene terpolymer-graft polystyrene (EPDM-graft PS) copolymer was successfully prepared by two steps of the polymerization of styrene via a RAFT process using dibenzyl trithiocarbonate (DBTTC), and of chain transfer of PS in the presence of dicumyl peroxide (DCP) as a radical generator, thus obtaining a graft copolymerer having 1 ~ 5 PS graft branches per EPDM.

The number of PS chains per EPDM and the composition thereof were adjusted by controlling two factors, namely, the amount of radical generator and the amount of PS. When the amounts of radical generator and PS were increased, the graft copolymer could have PS chains in higher concentration. When the amounts of radical generator and PS were increased, the grafting efficiency (GE) was increased. Because the molecular weight of PS can be easily

adjusted in the RAFT process, the above results show that all of three factors of the graft copolymer (i.e. the length of graft branch, the number of graft branches, and grafting efficiency) can be adjusted.

Although the embodiments of the present invention have been disclosed for illustrative purposes, those skilled in the art will appreciate that diverse variations and modifications are possible, without departing from the spirit and scope of the invention. Thus, the above embodiments should be understood not to be limited but to be illustrated. For example, respective elements described in an integrated form may be dividedly used, and the divided elements may be used in a state of being combined.

The scope of the present invention is defined by, rather than the above detailed description, the claims which will be described later, and all variations or modifications deduced from the meanings, scope and equivalents of the claims are intended to be included in the scope of the present invention.

**Industrial Applicability**

As described hereinbefore, the present invention provides a method of preparing an olefin-based segmented copolymer. According to the present invention, the olefin-based segmented copolymer can be easily synthesized using two simple steps.

Also according to the present invention, the amount of a radical generator and the amount of a vinyl-based polymer to be grafted are adjusted, thereby making it possible to control not only the length and number of graft chains of the resulting olefin-based segmented copolymer but also the grafting efficiency.

Thus, the use of the method according to the present invention enables the synthesis of olefin-based segmented copolymers having a variety of improved properties and wide applicability.

**Claims**

**1.** A method of preparing an olefin-based segmented copolymer, comprising:

polymerizing a vinyl-based monomer via reversible addition-fragmentation chain transfer (RAFT) using a trithiocarbonate-based chain transfer agent to synthesize a vinyl-based polymer; and
grafting the synthesized vinyl-based polymer onto an olefin-based polymer.

**2.** The method of claim 1, wherein the trithiocarbonate-based chain transfer agent is a compound represented by Chemical Formula 1 below:

[Chemical Formula 1]

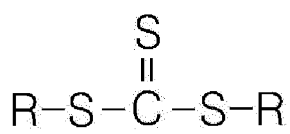

wherein two Rs are respectively independently selected from the group consisting of alkyl; alkenyl; saturated, unsaturated or aromatic carbocyclic or heterocyclic ring; alkylthio; alkoxy; and dialkylamino, and the alkyl; alkenyl; saturated, unsaturated or aromatic carbocyclic or heterocyclic ring; alkylthio; alkoxy; and dialkylamino may be independently substituted with a substituent selected from the group consisting of epoxy, hydroxy, alkoxy, aryl, acyl, acyloxy, carboxy and salts thereof, sulfonic acid and salts thereof, alkylcarbonyloxy, isocyanato, cyano, silyl, halo and dialkylamino.

**3.** The method of claim 2, wherein the trithiocarbonate-based chain transfer agent is dibenzyl trithiocarbonate (DBTTC).

**4.** The method of claim 1, wherein the vinyl-based monomer is selected from the group consisting of a compound represented by Chemical Formula 2 below and a combination thereof:

[Chemical Formula 2] $GH_2=CUV$

wherein U is selected from the group consisting of hydrogen; halogen; and $C_1$~$C_4$ alkyl which may be substituted with a substituent independently selected from the group consisting of hydroxy, $C_1$~$C_{18}$ alkoxy, aryloxy (OR'),

carboxy, acyloxy, aroyloxy ($O_2CR'$), alkoxy-carbonyl and aryloxy-carbonyl ($CO_2R'$), and
V is selected from the group consisting of hydrogen, R', $CO_2H$, $CO_2R'$, COR', CN, $CONH_2$, CONHR', $CONR'_2$, $O_2CR'$, OR' and halogen,
wherein R' is independently selected from the group consisting of $C_1$~$C_{18}$ alkyl, $C_2$~$C_{18}$ alkenyl, aryl, heterocyclyl, aralkyl and alkaryl, and the $C_1$~$C_{18}$ alkyl, $C_2$~$C_{18}$ alkenyl, aryl, heterocyclyl, aralkyl, and alkaryl may be substituted with a substituent independently selected from the group consisting of epoxy, hydroxy, alkoxy, aryl, acyl, acyloxy, carboxy and salts thereof, sulfonic acid and salts thereof, alkoxy- or aryloxy-carbonyl, isocyanato, cyano, silyl, halo, and dialkylamino.

**5.** The method of claim 4, wherein the vinyl-based monomer is selected from the group consisting of methyl methacrylate, ethyl methacrylate, propyl methacrylate, butyl methacrylate, 2-ethylhexyl methacrylate, isobornyl methacrylate, methacrylic acid, benzyl methacrylate, phenyl methacrylate, methacrylonitrile, alpha-methylstyrene, methyl acrylate, ethyl acrylate, propyl acrylate, butyl acrylate, 2-ethylhexyl acrylate, isobornyl acrylate, acrylic acid, benzyl acrylate, phenyl acrylate, acrylonitrile, styrene; functionalized methacrylate, acrylate and styrene selected from the group consisting of glycidyl methacrylate, 2-hydroxyethyl methacrylate, hydropropyl methacrylate, hydroxybutyl methacrylate, N,N-dimethylaminoethyl methacrylate, N,N-diethylaminoethyl methacrylate, triethyleneglycol methacrylate, itaconic anhydride, itaconic acid, glycidyl acrylate, 2-hydroxyethyl acrylate, hydroxypropyl acrylate, hydroxybutyl acrylate, N,N-dimethylaminoethyl acrylate, N,N-diethylaminoethyl acrylate, triethyleneglycol acrylate, methacrylamide, N-methylacrylamide, N,N-dimethylacrylamide, N-tert-butylmethacrylamide, N-n-butylmethacrylamide, N-methylolmethacrylamide, N-ethylolmethacrylamide, N-tert-butylacrylamide, N-n-butylacrylamide, N-methylolacrylamide, N-ethylolacrylamide, vinyl benzoic acid, diethylaminostyrene, alpha-methylvinyl benzoic acid, diethylamino alpha-methylstyrene, p-vinylbenzene sulfonic acid, sodium p-vinylbenzenesulfonate, trimethoxysilylpropyl methacrylate, triethoxysilylpropyl methacrylate, tributoxysilylpropyl methacrylate, dimethoxymethylsilylpropyl methacrylate, diethoxymethylsilylpropyl methacrylate, dibutoxymethylsilylpropyl methacrylate, diisopropoxymethylsilylpropyl methacrylate, dimethoxysilylpropyl methacrylate, diethoxysilylpropyl methacrylate, dibutoxysilylpropyl methacrylate, diisopropoxysilylpropyl methacrylate, trimethoxysilylpropyl acrylate, triethoxysilylpropyl acrylate, tributoxysilylpropyl acrylate, dimethoxymethylsilylpropyl acrylate, diethoxymethylsilylpropyl acrylate, dibutoxymethylsilylpropyl acrylate, diisopropoxymethylsilylpropyl acrylate, dimethoxysilylpropyl acrylate, diethoxysilylpropyl acrylate, dibutoxysilylpropyl acrylate, diisopropoxysilylpropyl acrylate, vinyl acetate, vinyl butyrate, vinyl benzoate, vinyl chloride, vinyl fluoride, vinyl bromide, maleic anhydride, N-phenylmaleimide, N-butylmaleimide, N-vinylpyrrolidone, N-vinylcarbazole, butadiene, isoprene, chloroprene and propylene; and combinations thereof.

**6.** The method of claim 1, wherein the synthesized vinyl-based polymer is selected from the group consisting of polystyrene, polyacrylate, polymethacrylate and polyacrylonitrile.

**7.** The method of claim 1, wherein the olefin-based polymer is selected from the group consisting of polyethylene, polypropylene, polybutadiene, an ethylene-propylene-diene terpolymer and combinations thereof.

**8.** The method of claim 1, wherein the grafting is performed in presence of a radical generator for generating a reactive radical site on the olefin-based polymer.

**9.** The method of claim 8, wherein an amount of the radical generator and an amount of the synthesized vinyl-based polymer are adjusted, whereby a number of vinyl-based polymer chains to be grafted onto the olefin-based segmented copolymer and grafting efficiency are adjusted.

**10.** The method of claim 1, wherein the grafting is performed in a solution phase in a solvent.

**11.** The method of claim 1, wherein the grafting is performed in a melt phase.

**12.** An olefin-based segmented copolymer, prepared using the method of any one of claims 1 to 11.

[FIG. 1]

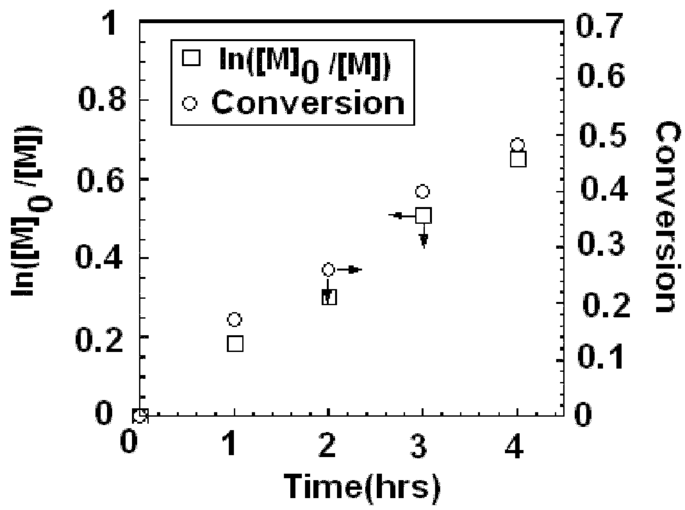
ln([M]0 /[M])
Conversion
ln([M]0 /[M])
Conversion
Time(hrs)

(a)

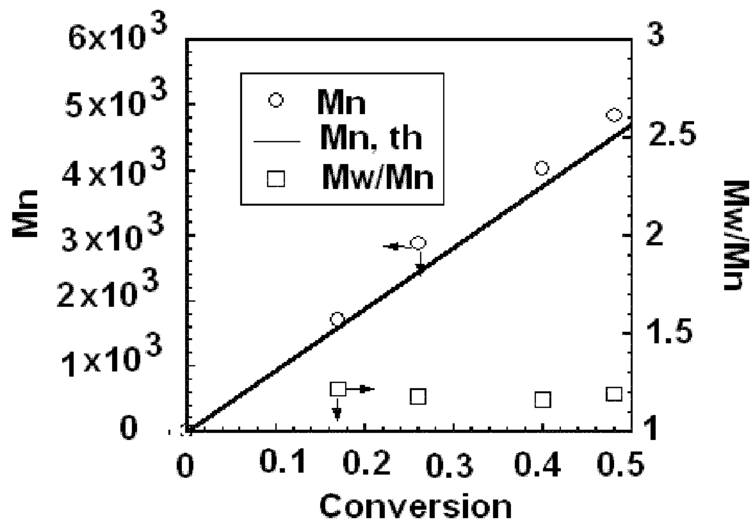
Mn
Mn, th
Mw/Mn
Mn
Mw/Mn
Conversion

(b)

[FIG. 2]

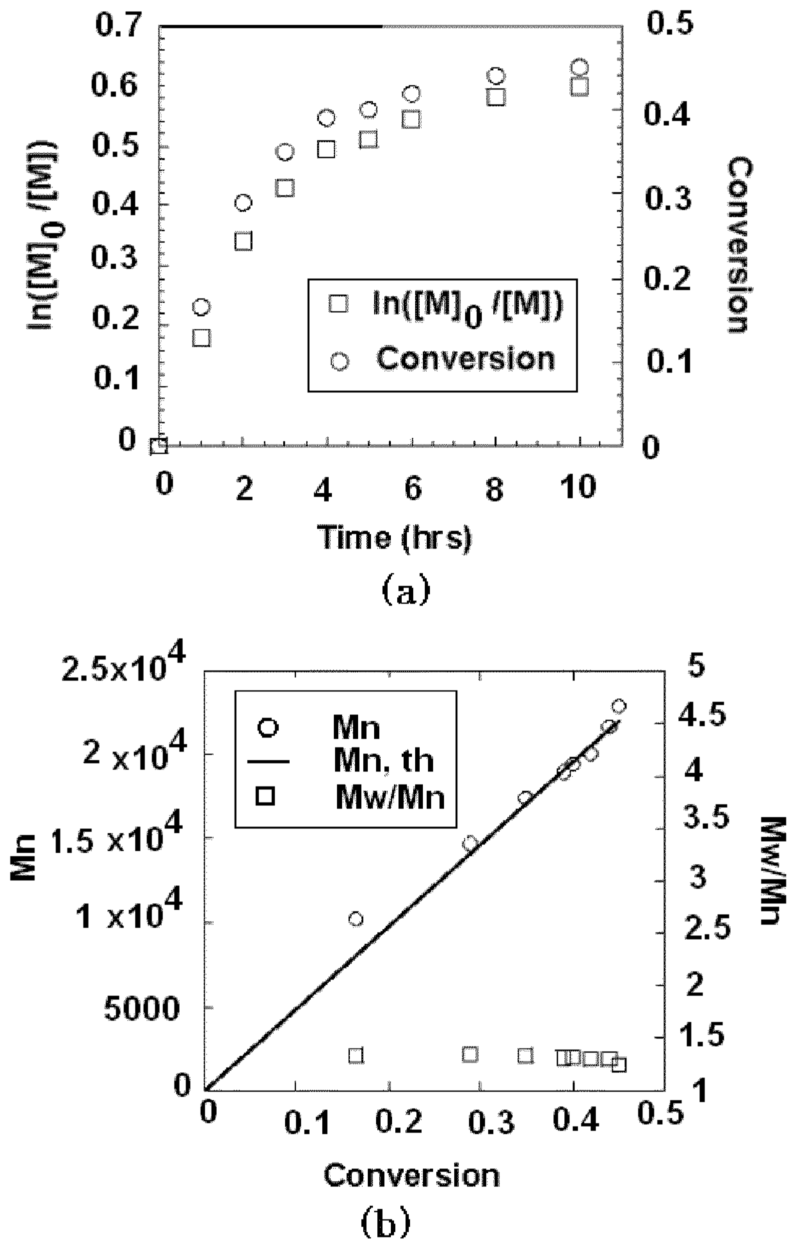
0.7
0.6
0.5
0.4
0.3
0.2
0.1
0
ln([M]0 /[M])
0.5
0.4
0.3
0.2
0.1
0
Conversion
ln([M]0 /[M])
Conversion
0
2
4
6
8
10
Time (hrs)
(a)
2.5x10^4
2 x10^4
1.5 x10^4
1 x10^4
5000
0
Mn
5
4.5
4
3.5
3
2.5
2
1.5
1
Mw/Mn
Mn
Mn, th
Mw/Mn
0
0.1
0.2
0.3
0.4
0.5
Conversion
(b)

[FIG. 3]

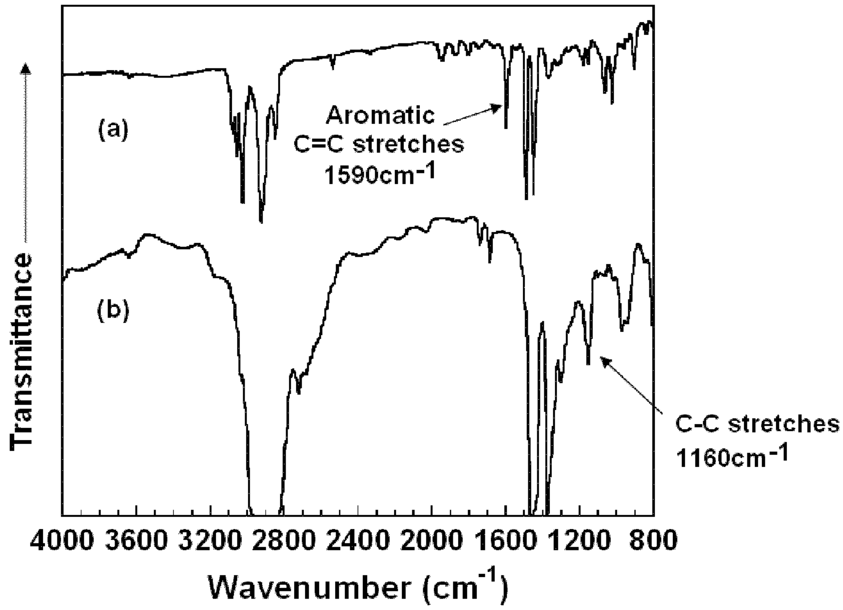
Transmittance
(a)
(b)
Aromatic
C=C stretches
1590cm-1
C-C stretches
1160cm-1
4000 3600 3200 2800 2400 2000 1600 1200 800
Wavenumber (cm-1)

[FIG. 4]

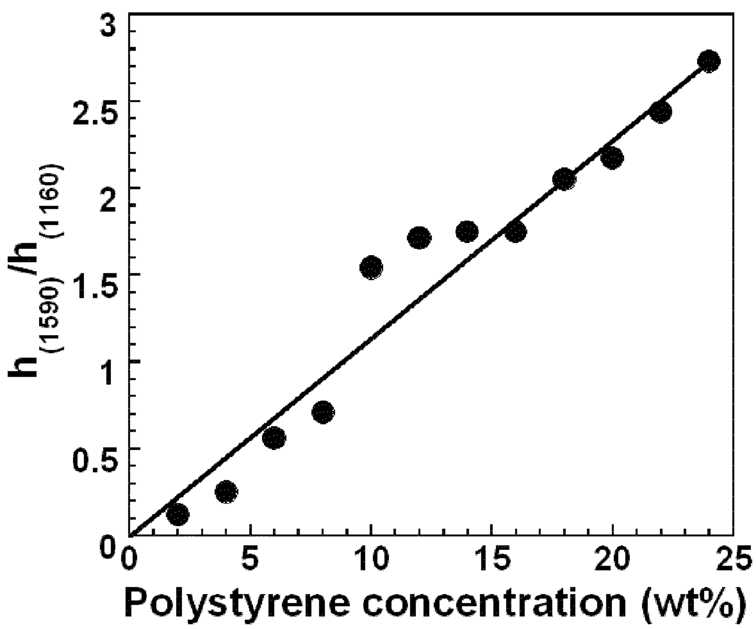
h(1590)/h(1160)
0
0.5
1
1.5
2
2.5
3
0
5
10
15
20
25
Polystyrene concentration (wt%)

[FIG. 5]

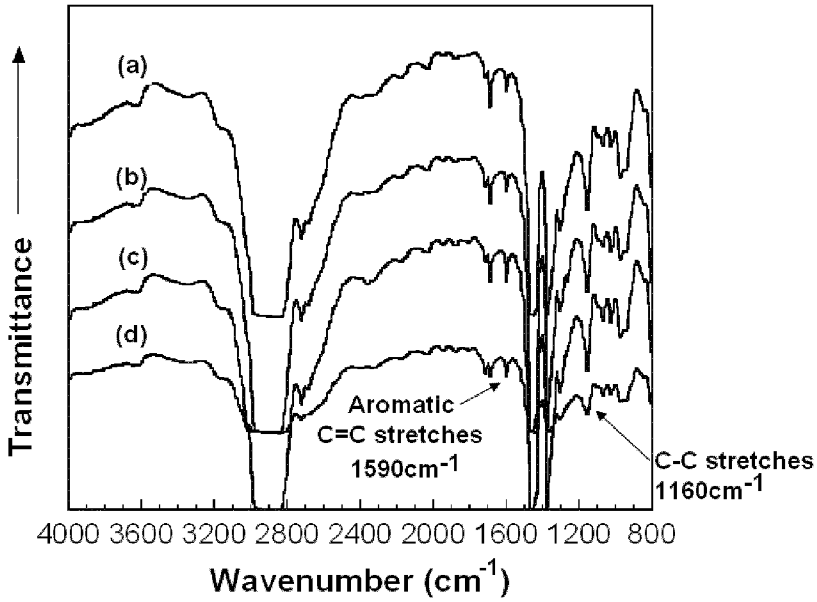
(a)
(b)
(c)
(d)
Transmittance
Aromatic
C=C stretches
1590cm-1
C-C stretches
1160cm-1
4000 3600 3200 2800 2400 2000 1600 1200 800
Wavenumber (cm-1)

[FIG. 6]

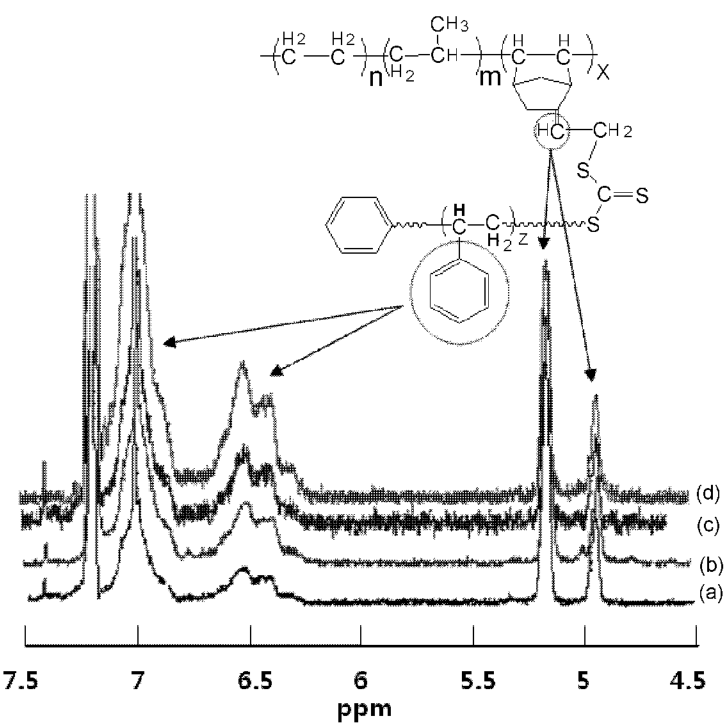
(d)
(c)
(b)
(a)
7.5 7 6.5 6 5.5 5 4.5
ppm

[FIG. 7]

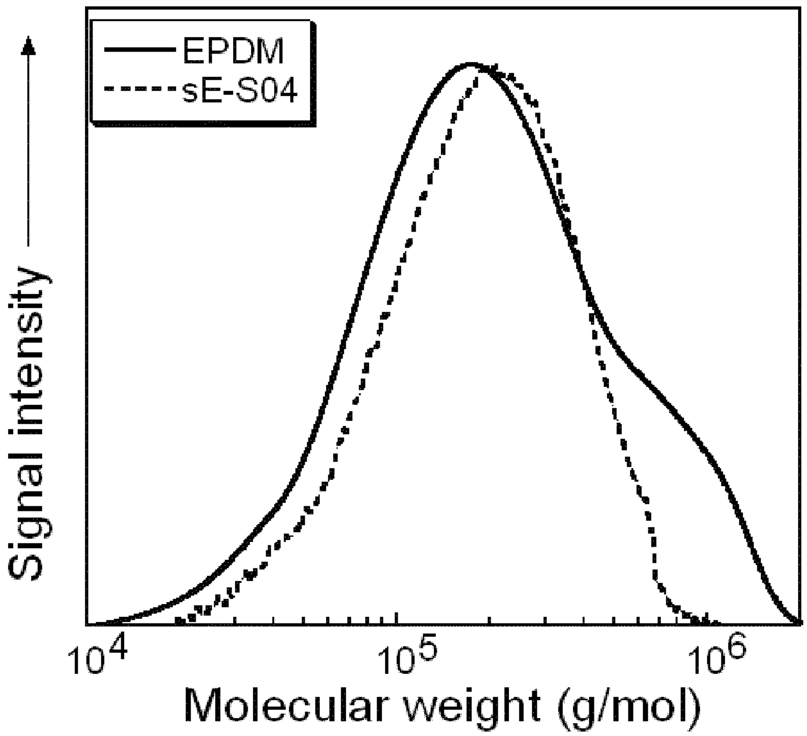
EPDM
sE-S04
Signal intensity
$10^4$
$10^5$
$10^6$
Molecular weight (g/mol)

[FIG. 8]

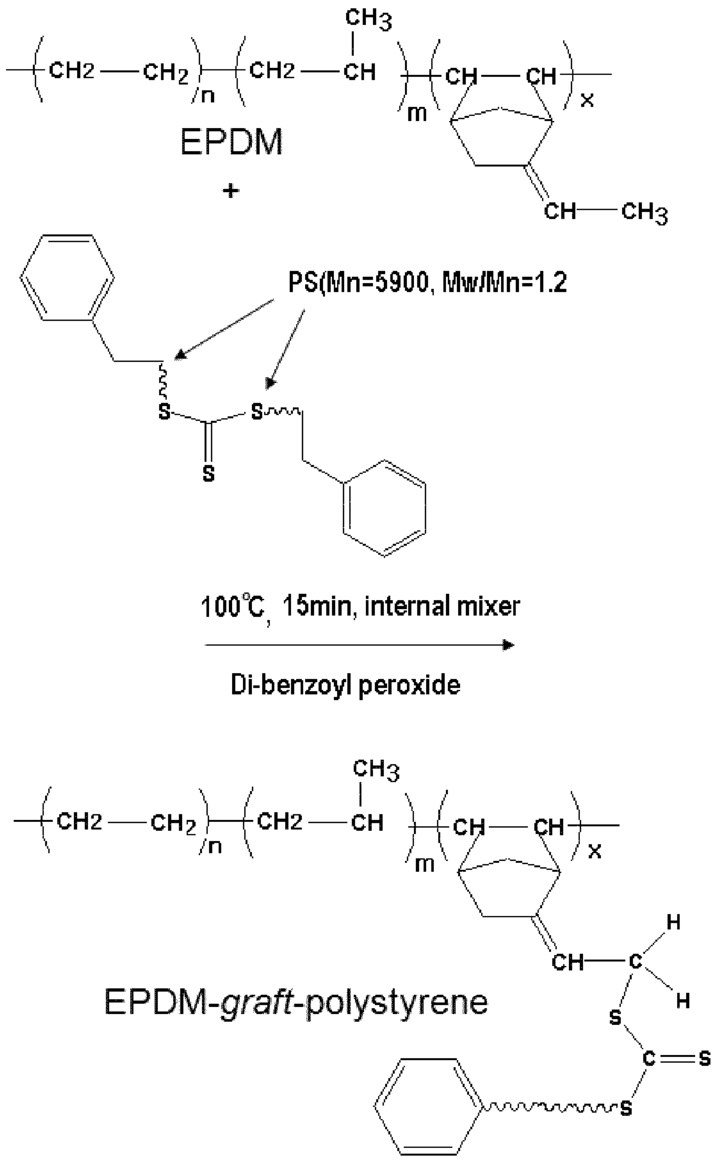
EPDM
+
PS(Mn=5900, Mw/Mn=1.2
100℃, 15min, internal mixer
Di-benzoyl peroxide
EPDM-*graft*-polystyrene